# EUROPEAN PATENT APPLICATION

(11) **EP 3 034 495 A1**
(43) Date of publication of application: **22.06.2016**
(21) Application number: 14836674.3
(22) Date of filing: 28.07.2014
(51) Int. Cl.: C07D 209/20, C07K 5/078, G01N 27/62

(54) **COMPOUND FOR LABELLING SUGAR CHAIN SAMPLE**

(30) Priority: 16.08.2013 JP 2013169090
(71) Applicant: Sumitomo Bakelite Co.,Ltd., Tokyo 140-0002 (JP)
(72) Inventor: ABE, Hiroki, Tokyo 140-0002 (JP); SHIMAOKA, Hideyuki, Tokyo 140-0002 (JP)
(74) Representative: Müller-Boré & Partner Patentanwälte PartG mbB
(86) International application number: PCT/JP2014/069804
(87) International publication number: WO 2015/022854

(57) **Abstract**

A compound having the following structure has been found out: 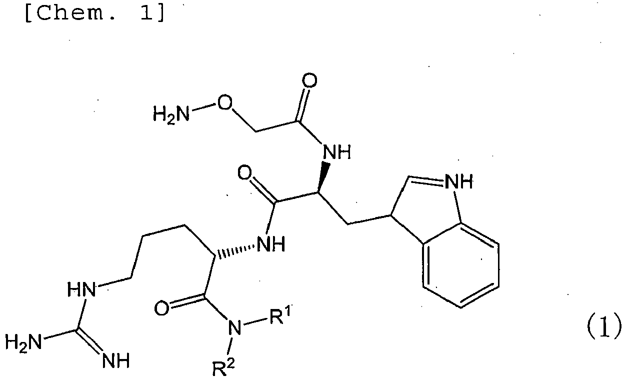 (R¹ and R² each independently represent any one of a hydrogen atom and an alkyl group).

## Description

### [Technical Field]

The present invention relates to a novel compound for labeling a sugar chain sample, as well as a method for preparing a sugar chain sample and a method for analyzing a sugar chain which utilize the compound.

### [Background Art]

Biological polymers such as sugar chains, glycoproteins, glycopeptides, peptides, oligopeptides, proteins, nucleic acids, and lipids, play important roles in biotechnology fields including medical science, cell engineering, organ engineering, and so forth. The elucidation of the control mechanism of biological reactions using these substances leads to developments in the biotechnology fields.

Sugar chain is a generic term for molecules composed of monosaccharides, such as glucose, galactose, mannose, fucose, xylose, N-acetylglucosamine, N-acetylgalactosamine, and sialic acid, and derivatives thereof, which are linked via a glycosidic bond to give a chain form. Among biological polymers, sugar chains are very rich in diversity. It is about to be revealed that sugar chains are closely associated with various functions of naturally-occurring organisms, for example, intercellular communications, regulations of protein functions and interaction, and so forth.

Sugar chains often exist *in vivo* in a form of glycoconjugate linked to a protein, a lipid, or the like. Examples of a biological polymer having a sugar chain include: proteoglycans in plant cell walls contributing to stabilization of the cells; glycolipids influencing cell differentiation, proliferation, adhesion, migration, and so on; glycoproteins associated with intercellular interaction and cell recognition; and the like. There have been gradually revealed mechanisms of how sugar chains contained in these biological polymers control biological reactions in a highly precise manner while substituting, assisting, enhancing, regulating, or inhibiting functions mutually with the biological polymers. If involvements of such sugar chains in cell differentiation and proliferation, cell adhesion, immunity, and neoplastic transformation of cells are elucidated, a new development can be expected by closely relating this sugar chain engineering to medical science, cell engineering, or organ engineering.

In glycoprotein drugs, sugar chains play important roles in expressions of biological activity, and the like, in many cases. Thus, evaluating the sugar chains is quite important as a parameter in quality control of glycoprotein drugs. Particularly, it has been reported that the structure of a sugar chain in an antibody drug determines the antibody-dependent cell-mediated cytotoxicity activity (ADCC activity). Since then, the importance of analyzing the structure of sugar chain has been increasing.

Hence, recently, a method for analyzing a sugar chain structure in a rapid, simple, and precise manner has been demanded. Accordingly, sugar chain analyses have been conducted by a wide variety of methods such as high-performance liquid chromatography (HPLC), nuclear magnetic resonance spectroscopy, capillary electrophoresis (CE method), mass spectrometry, and lectin array method.

For the sugar chain analyses using these various methods, it is necessary to separate and purify in advance a sugar chain from a protein, peptide, lipid, nucleic acid, or the like contained in a biological sample. However, such purification and labeling of a sugar chain require time and man-hour, so that it is difficult to prepare a large amount of various samples at a time.

As a technique for solving this problem, a method has been reported by which a sugar chain sample is prepared by utilizing a sugar chain-capturing substance having a functional group (for example, a hydrazide group or an aminooxy group) capable of specifically reacting with an aldehyde group of a sugar chain (PTL 1) . In this method, the sugar chain captured by the sugar chain-capturing substance is released and labeled for the analysis by mass spectrometry or high-performance liquid chromatography. In addition, particularly, in the case of mass spectrometry, a compound (N-Aminooxyacetyl-tryptophyl-(arginine methyl ester); hereinafter referred to as "aoWR") having the following structure is used as a labeling reagent to increase the ionization efficiency of a sugar chain, which is the analysis target.

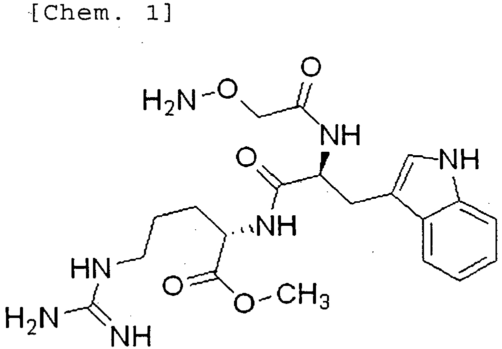

The aoWR has an aminooxy group at a terminal end thereof, and this aminooxy group involves in a hydrazone bond or an oxime bond between the sugar chain-capturing substance and the sugar chain. Moreover, the sugar chain is released from the sugar chain-capturing substance by a hydrazone-oxime exchange reaction or an oxime-oxime exchange reaction, and simultaneously the aoWR can bind to the sugar chain. Hence, there is an advantage of enabling efficient sugar-chain labeling.

### [Citation List]

### [Patent Literature]

[PTL 1] International Publication No. WO2008/018170

### [Summary of Invention]

### [Technical Problems]

An object of the present invention is to provide a labeling compound enabling a more efficient analysis in mass spectrometry analysis of a sugar chain sample prepared by utilizing a sugar chain-capturing substance. Another object of the present invention is to provide a method for preparing a sugar chain sample and a method for analyzing a sugar chain which utilize the labeling compound.

### [Solution to Problems]

The present inventors have earnestly studied to achieve the above objects. As a result, the inventors have found out compounds having the following structure.

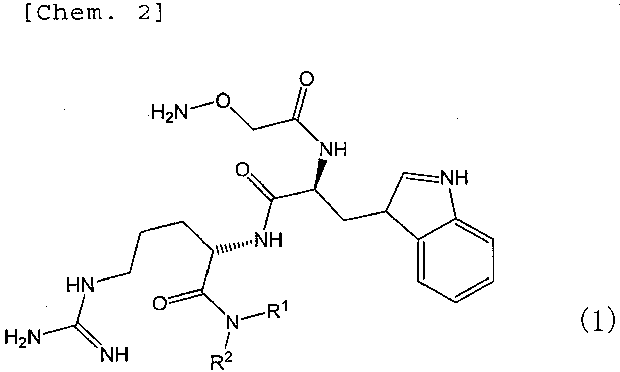

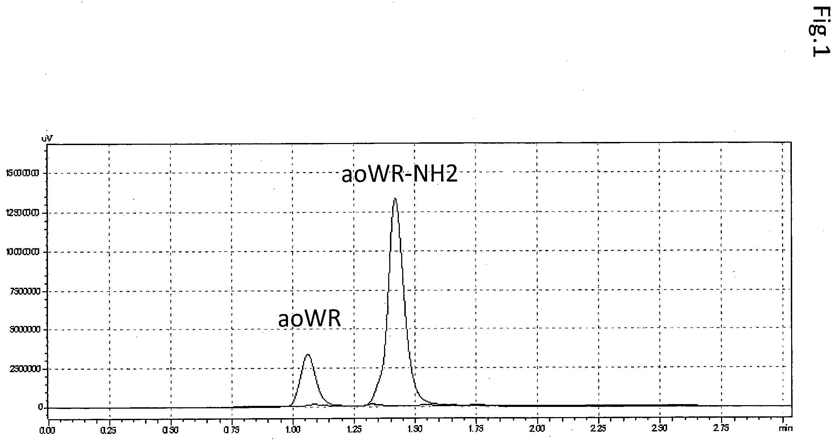

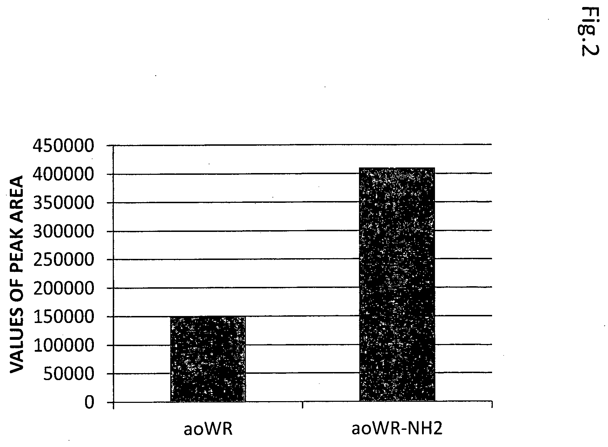

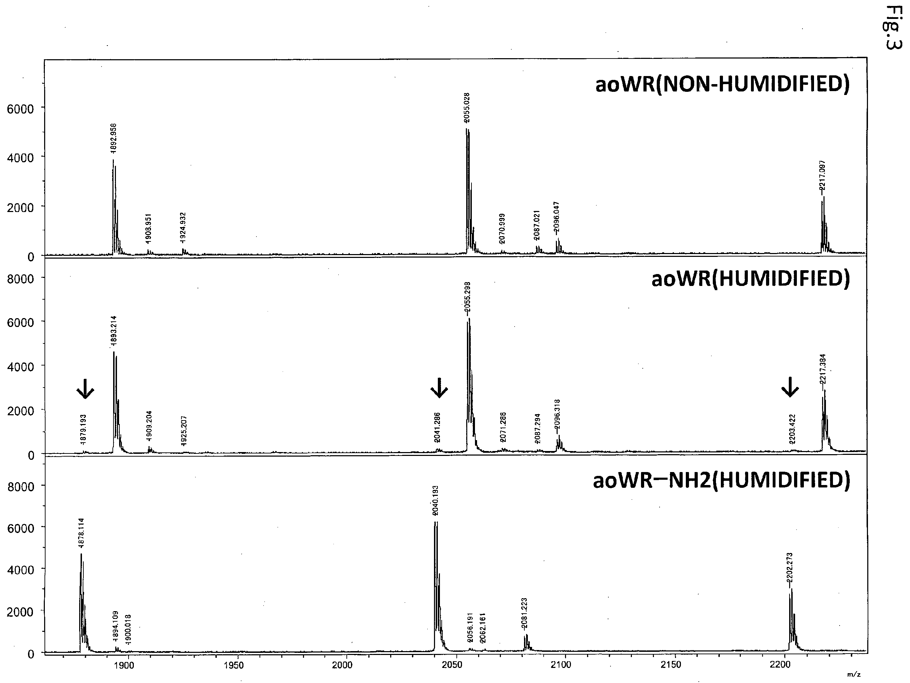

In the formula (1), R¹ and R² each independently represent any one of a hydrogen atom and an alkyl group. The compounds are conventional aoWR in which a methyl ester (-CO-CH₃) group is converted to an amide group (-CO-NR¹R²). Measuring the fluorescence intensities revealed that the compound in which, for example, the amide group was -CO-NH₂ (N-Aminooxyacetyl-tryptophyl-(arginine amide); hereinafter referred to as "aoWR-NH2") exhibited a significant fluorescence intensity approximately 2.7 times as high as that of aoWR (Figs. 1 and 2). Moreover, in a case where humidified aoWR was used as a labeling reagent in preparing a sugar chain sample using a sugar chain-capturing substance, when a sugar chain was detected by mass spectrometry, peaks of degradation products of the labeling reagent appeared near peaks of the sugar chain. In contrast, in a case where, for example, aoWR-NH2 was used among the above-described compounds, no peak of such a degradation product appeared (Fig. 3).

From the foregoing, the present inventors have found that the compound represented by the formula (1) not only improves the ionization efficiency but is also excellent in fluorescence intensity and storage stability, and that the compound is very useful inpreparing a sugar chain sample and detecting a sugar chain sample by using a sugar chain-capturing substance. These discoveries have led to the completion of the present invention.

Thus, the present invention relates to a novel compound represented by the above formula (1), a labeling reagent comprising the compound as an effective component, and a method for preparing a sugar chain sample and a method for analyzing a sugar chain which utilize the compound. More specifically, the present invention provides the following inventions.
[1] A compound represented by the following formula (1) : 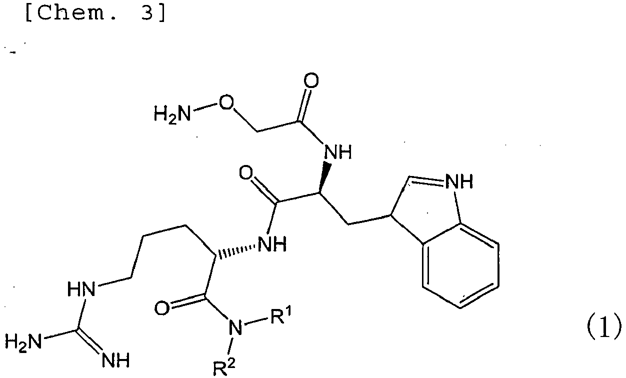 (R¹ and R² each independently represent any one of a hydrogen atom and an alkyl group).
[2] The compound according to [1], wherein R¹ and R² in the formula (1) each independently represent any one of a hydrogen atom, a methyl group, and an ethyl group.
[3] The compound according to [1] or [2], which is represented by the following formula: 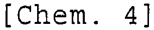 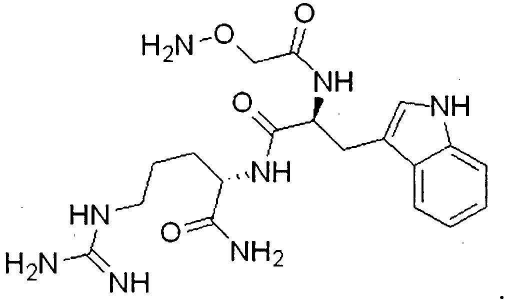
[4] A labeling reagent comprising the compound according to anyone of [1] to [3] as an effective component.
[5] A method for preparing a labeled sugar chain sample, comprising the steps of:
   (a) bringing a sample containing a sugar chain into contact with a carrier having any one of a hydrazide group and an aminooxy group as a functional group for capturing the sugar chain to cause an aldehyde group of the sugar chain to react with the any one of a hydrazide group and an aminooxy group of the carrier, thereby capturing the sugar chain on the carrier;
   (b) washing the carrier capturing the sugar chain; and
   (c) bringing the compound according to any one of [1] to [3] into contact with the carrier capturing the sugar chain to release the sugar chain from the carrier and simultaneously to bind the compound to the sugar chain.
[6] The method according to [5], wherein the carrier is coated with a polymer substance having a crosslinked polymer structure represented by the following formula (2) : 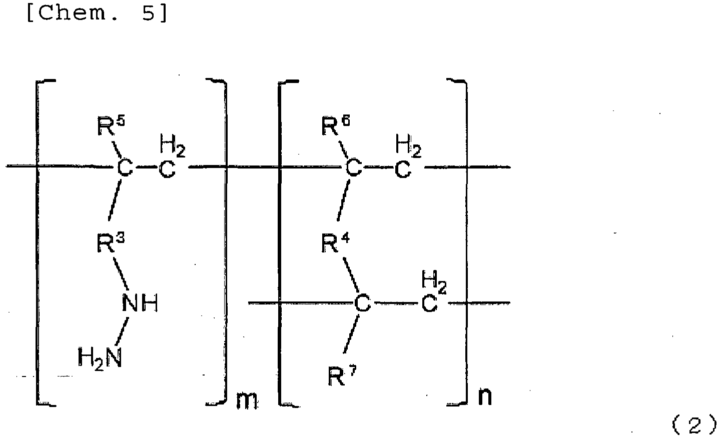 (R³ and R⁴ each independently represent a hydrocarbon chain having 1 to 20 carbon atoms which may be interrupted with any one of -O-, -S-, -NH-, -CO-, and -CONH-; R⁵, R⁶, and R⁷ each independently represent any one of H, CH₃, and a hydrocarbon chain having 2 to 5 carbon atoms; and m and n each independently represent the number of monomer units).
[7] The method according to [6], wherein the carrier is coated with a polymer substance having a crosslinked polymer structure represented by the following formula (3): 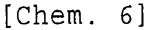 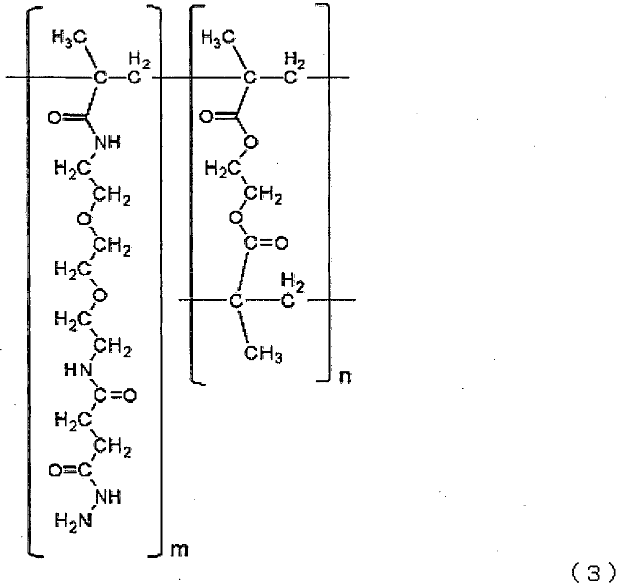 (m and n each independently represent the number of monomer units).
[8] A method for analyzing a sugar chain, comprising the step of subj ecting a labeled sugar chain sample prepared by the method according to any one of [5] to [7] to mass spectrometry to detect a sugar chain.

### [Advantageous Effects of Invention]

The present invention makes it possible to efficiently prepare a labeled sugar chain from an unpurified biological sample containing a lot of contaminants, and to efficiently detect the prepared labeled sugar chain by mass spectrometry.

### [Brief Description of Drawings]

[Fig. 1] Fig. 1 is a graph showing fluorescence peaks of aoWR-NH2 and aoWR detected as a result of detecting fluorescence intensities of the aoWR-NH2 and the aoWR using a HPLC system.
[Fig. 2] Fig. 2 is a graph showing values of the fluorescence peak areas of the aoWR-NH2 and the aoWR detected in Fig. 1.
[Fig. 3] Fig. 3 shows graphs for illustrating the result of detecting a sugar chain sample labeled with aoWR-NH2 or aoWR by mass spectrometry (MALDI-TOF MS). The result of detecting non-humidified aoWR is shown at the top of the figure, the result of detecting humidified aoWR is shown at the middle of the figure, and the result of detecting humidified aoWR-NH2 is shown at the bottom of the figure.

### [Description of Embodiments]

The present invention provides a novel compound represented by the following formula (1):

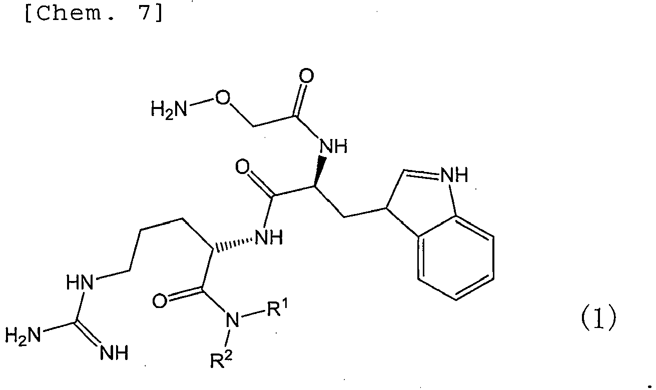

In the formula (1), R¹ and R² each independently represent any one of a hydrogen atom and an alkyl group. In the case where the R¹ and/or R² are each an alkyl group, the ionization efficiency is high. Accordingly, when a sugar chain sample labeled with the compound of the present invention is subjected to mass spectrometry, it tends to be easier to conduct the analysis by MALDI-TOF MS. The alkyl group is preferably a methyl group or an ethyl group. On the other hand, in the case where the R¹ and/or R² are each a hydrogen atom, the synthesis is possible using a common commercially-available reagent as it is. Accordingly, the production process is readily shortened, and the cost reduction is possible.

In the compound of the present invention, both R¹ and R² are preferably hydrogen atoms from the viewpoint of cost reduction. The compound of the present invention is preferably a compound designated as aoWR-NH2 having the following structure:

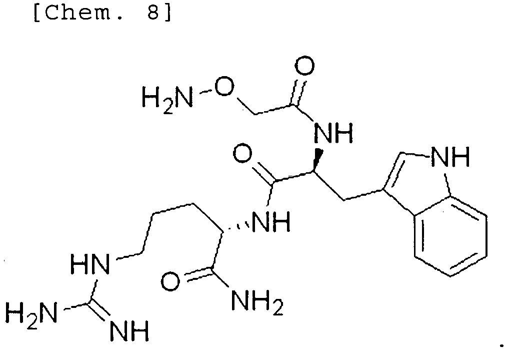

The compound of the present invention represented by the formula (1), when binding to a sugar chain, exhibits an effect of increasing the ionization efficiency of the sugar chain in mass spectrometry, and further can also exhibit quite a high fluorescence intensity as described in the present Example 1. Surprisingly, the fluorescence intensity of the compound of the present invention itself, for example, aoWR-NH2, is approximately 2.7 times as high as that of conventional aoWR. Thus, the present invention also provides a labeling reagent comprising the compound of the present invention as an effective component, for increasing the ionization efficiency or imparting fluorescent characteristics.

The compound of the present invention can be used in a method for preparing a sugar chain by using a carrier having a functional group for capturing the sugar chain, so that the sugar chain captured on the carrier can be released and labeled. Thus, the present invention provides a method for preparing a labeled sugar chain sample, comprising the steps of: (a) bringing a sample containing a sugar chain into contact with a carrier having any one of a hydrazide group and an aminooxy group as a functional group for capturing the sugar chain to cause an aldehyde group of the sugar chain to react with the any one of a hydrazide group and an aminooxy group of the carrier, thereby capturing the sugar chain on the carrier; (b) washing the carrier capturing the sugar chain; and (c) bringing the compound of the present invention into contact with the carrier capturing the sugar chain to release the sugar chain from the carrier and simultaneously to bind the compound of the present invention to the sugar chain, thereby obtaining a sugar chain sample labeled with the compound of the present invention.

As the "carrier" used in the step (a), polymer particles are preferably used. The polymer particles, which are either solid particles or gel particles, make it possible to readily recover a sugar chain captured on the polymer particles by means such as centrifugation or filtration. Meanwhile, the polymer particles can also be used while filled in a column. The method using the polymer particles filled in a column is important particularly from the viewpoint of successive operations. The use of a filter plate (for example, MultiScreen Solvinert Filter Plate manufacturedbyMillipore Corporation) as a reaction vessel enables simultaneous treatment on multiple samples. Further, the throughput of the sugar chain purification is greatly improved in comparison with conventional purificationmeans, for example, using a column represented by gel filtration. Alternatively, the use of magnetic beads as the particles makes it possible to collect the beads on the vessel wall surface and the like utilizing a magnetic force, so that the beads can be washed readily.

The shape of the polymer particles is not particularly limited, but the shape is preferably spherical or similar shapes. In the case of spherical polymer particles, the average particle diameter is preferably 0.05 to 1000 µm, more preferably 0.05 to 200 µm, furthermore preferably 0.1 to 200 µm, and most preferably 0.1 to 100 µm. If the average particle diameter is below the lower limit value, it becomes necessary to apply a high pressure to compensate for poor passage of the solution through a column filled with the polymer particles thus used. In addition, it is also difficult to recover the polymer particles by centrifugation or filtration. If the average particle diameter exceeds the upper limit value, the contact area between the polymer particles and the sample solution is small, and the efficiency of capturing the sugar chain is decreased.

The carrier has any one of a hydrazide group and an aminooxy group as a functional group for capturing a sugar chain at least on a part of the surface. These functional groups are capable of reacting with an aldehyde group of a sugar chain, thereby capturing the sugar chain.

As the carrier for capturing the sugar chain, it is particularly preferable to use a crosslinked polymer (polymer substance) having a crosslinked polymer structure represented by the following formula (2) or formula (3), or a carrier coated with the polymer substance. 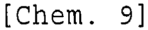 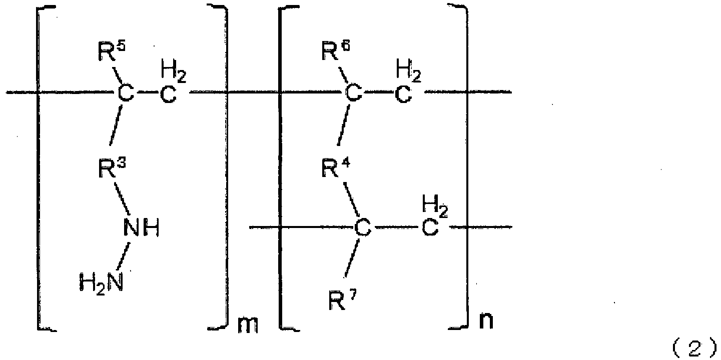 (R³ and R⁴ each independently represent a hydrocarbon chain having 1 to 20 carbon atoms which may be interrupted with any one of -O-, -S-, -NH-, -CO-, and -CONH-; R⁵, R⁶, and R⁷ each independently represent any one of H, CH₃, and a hydrocarbon chain having 2 to 5 carbon atoms; and m and n each independently represent the number of monomer units.)

The example of the crosslinked polymer includes a crosslinked polymer having the following structure: 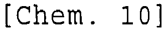 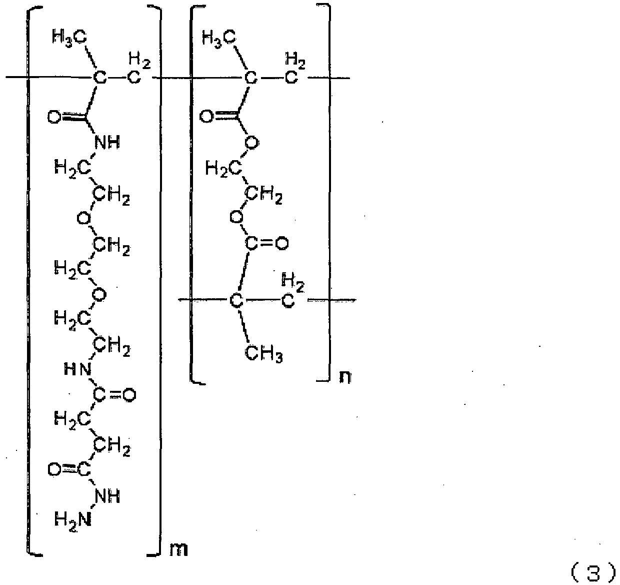 (m and n each independently represent the number of monomer units).

In the present invention, "BlotGlyco^{(R)}" (manufactured by Sumitomo Bakelite Co., Ltd., #BS-45603), which is hydrazide group- containing polymer particles, can be suitably used.

When a sugar chain is captured with the polymer particles for capturing the sugar chain, the pH of the reaction system is preferably 2 to 9, more preferably 2 to 7, and furthermore preferably 2 to 6. For the pH adjustment, various buffer solutions can be used. The temperature during the capturing of the sugar chain is preferably4 to 90°C, more preferably 4 to 70°C, furthermore preferably 30 to 80°C, and most preferably 40 to 80°C. The reaction time can be set as appropriate. The sample solution may be passed through a column filled with the polymer particles.

Washing the carrier capturing the sugar chain in the step (b) enables the removal of substances (non-specifically adsorbed substances) other than the sugar chain among substances captured with the carrier.

As the method for removing the substances other than the sugar chain, it is possible to adopt: a washing method using an aqueous solution of guanidine, which is a chaotropic agent having an ability to dissociate a hydrophobic bond; or a washing method using pure water or an aqueous buffer solution (for example, a phosphate buffer solution, a tris buffer solution, or the like). As the washing conditions in the washing step, the temperature is 4 to 40 °C, and the washing time is 10 seconds to 3 0 minutes. As the washing method, polymer particles as the carrier is immersed in a washing liquid, and the washing liquid is repeatedly replaced, so that the carrier can be washed.

To be more specific, the polymer particles are introduced in a centrifuge tube or a tube, and a washing liquid is added therein and shaken. Then, the polymer particles are precipitated by a centrifugation operation, and the supernatant is removed. These operations are repeated for the washing. For example, polymer particles are introduced in a centrifuge tube, and a washing liquid is added therein. After the polymer particles are spontaneously precipitated or forcibly precipitated by centrifugation, the supernatant is removed. By repeating these operations, the carrier can be washed. The washing operations are preferably performed 3 to 6 times. In the case of using magnetic beads, the centrifugation operation is not necessary, and the process is thus simple.

Moreover, it is also possible to use a tubular vessel which is a filter tube with a bottom surface portion provided with a filter having such a pore diameter that a liquid can pass therethrough but the beads cannot pass therethrough. The use of the filter tube with the polymer particles put therein enables the removal of a washing liquid required for the washing through the filter, and eliminates the need for the supernatant removal step after the centrifugation operation, so that the workability can be improved.

Further, various multiwell plates having 6 to 384 wells with bottom portions provided with the filter are commercially available. The use of these plates can increase the throughput. Particularly, for a multiwell plate having 96 wells, solution dispensing instruments, suction removal systems, plate conveyance systems, and so forth have been developed, and such a 96-well plate is optimal for increasing the throughput.

When the sugar-chain capturing reaction is successively carried out, the washing treatment may be performed successively after the sugar-chain capturing reaction by passing a washing solution through a column. Meanwhile, in the case of using a multiplate, substances other than the carrier capturing the sugar chain may be removed by a filtration operation or a centrifugation operation.

Note that excessive functional groups on the carrier can be capped by utilizing, for example, acetic anhydride or the like.

In the step (c), the compound of the present invention is brought into contact with the carrier capturing the sugar chain to release the sugar chain from the carrier and simultaneously to bind the compound of the present invention to the sugar chain. In the case where a carrier having a hydrazide group is used as the carrier for capturing the sugar chain, this step is carried out by a hydrazide-oxime exchange reaction as an aminooxy group present at a terminal end of the compound of the present invention acts on a hydrazone bond between the carrier and the sugar chain. In the case where a carrier having an aminooxy group is used as the carrier for capturing the sugar chain, this step is carried out by an oxime-oxime exchange reaction as the aminooxy group present at the terminal end the compound of the present invention acts on an oxime bond between the carrier and the sugar chain. In this manner, the compound of the present invention makes it possible to release the sugar chain from the carrier and to label the sugar chain by a single treatment.

As the pH of the reaction solution during the exchange reaction, the pH of 2 to 7 is preferable, the pH of 3 to 6 is more preferable, and the pH of 3.5 to 5.5 is most preferable. By adding an acetic acid/acetonitrile solution, the reaction solution can be adjusted to the above pH. The temperature during the exchange reaction is preferably 50 to 95°C, more preferably 60 to 90°C, and most preferably 70 to 90°C. During the exchange reaction, the reaction vessel is opened for the heat treatment. Thereby, the reaction proceeds while the solvent is being evaporated. Finally, the resultant is solidified to dryness. Thus, the exchange reaction can be carried out efficiently.

The analysis by analysis means such as mass spectrometry may be conducted on the recovered labeled sugar chain solution as it is, or after the excessively-contained labeling compounds are removed.

Additionally, the present invention provides a method for analyzing a sugar chain, comprising the step of subjecting a labeled sugar chain sample prepared by the above-described method to mass spectrometry to detect a sugar chain. As described in Example 2, when a sugar chain labeled with the compound of the present invention

(aoWR-NH2) is subjected to mass spectrometry, no peak of a degradation product of the aoWR-NH2 is detected near peaks of the sugar chain. This makes it possible to efficiently detect the sugar chain by utilizing mass spectrometry. As the mass spectrometry method, for example, MALDI-TOF MS can be suitably utilized. A mass spectrum obtained as a result of the mass spectrometry can be analyzed using analysis software or the like. Peaks of the sample sugar chain can be detected by reading the mass-to-charge ratio (m/z value). Further, the quantification of the sugar chain and the structural analysis of the sugar chain can be conducted based on the mass-to-charge ratio and the peak intensity (any indicator such as peak height or peak area). In the analysis of a sugar chain, various databases (for example, GlycoSuite and the like) can be utilized.

### [Examples]

Hereinafter, the present invention will be described more specifically based on Synthesis Example, Examples, and Comparative Examples. However, the present invention is not limited to the following Synthesis Example and Examples.

### (Synthesis Example 1) Synthesis of aoWR-NH2

The synthesis scheme is described below. 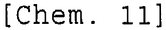 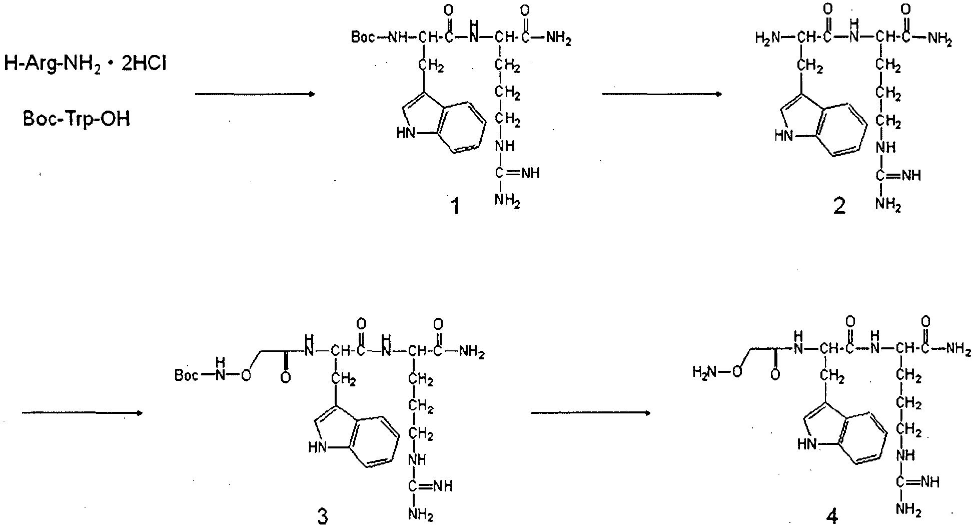 THF (40 mL) was added to Boc-Trp-OH (Merck KGaA: 853038, 4.66 g), and the solution was cooled to -50°C. To the solution, 4-methylmorpholine (1.86 g) and isobutyl chloroformate (2.51 g) were added and stirred for 10 minutes at -40° C and then for 30 minutes while the temperature was being raised to 0 °C. Thereby, an acid anhydride mixture (A) was prepared. Next, methanol (40 mL) was added to H-Arg-NH2·2HCl (Watanabe Chemical Industries, Ltd.: K00125, 4.53 g) and cooled to 5°C, and triethylamine (1.86 g) was added thereto. The solution thus prepared was added to (A). The resultant was stirred for 10 minutes at 0°C and then for 90 minutes while the temperature was being raised to room temperature. The reaction solution was concentrated under a reduced pressure. Thus, a compound 1 (15.96 g) was obtained.

To the compound 1 (15.96 g), methanol (26 mL) was added and cooled to 0 °C. Subsequently, a 4 M solution of HCl/dioxane (26 mL) was added thereto. The resultant was stirred for 30 minutes at 0°C and then for 3 hours while the temperature was being raised to room temperature. The reaction solution was concentrated under a reduced pressure. Thus, a compound 2 (13.82 g) was obtained.

THF (40 mL) was added to Boc-amino-oxyacetic acid (Merck KGaA: 851017, 2.45 g), and the solution was cooled to -40°C. To the solution, 4-methylmorpholine (1.55 g) and isobutyl chloroformate (2.09 g) were added and stirred for 15 minutes at -30°C and then for 35 minutes while the temperature was being raised to 0°C. Thereby, an acid anhydride mixture (B) was prepared. Next, distilled water (20 mL) was added to the compound 2 (13.82 g) and cooled to 5°C, and sodium hydrogen carbonate (1.29 g) was added thereto. The solution thus prepared was added to (B). The resultant was stirred for 1 hour at 5°C and then for 6 hours while the temperature was being raised to room temperature. The reaction solution was concentrated under a reduced pressure. Subsequently, the resultant was purified by ODS medium pressure chromatography. Thus, a compound 3 (4.82 g) was obtained.

To the compound 3 (4.82 g), methanol (14.5 mL) was added and cooled to 0°C. Subsequently, a 4 M solution of HCl/dioxane (14.5 mL) was added thereto. The resultant was stirred for 2 hours while the temperature was being raised to room temperature. The reaction solution was concentrated under a reduced pressure. Of 3.96 g of the obtained residue, 3.2 g was purified by ODS medium pressure chromatography. Thus, a compound 4 (aoWR-NH2, 1.2 9 g) was obtained.

### (Example 1)

HPLC was carried out on 20 mM aoWR and 20 mM aoWR-NH2 using Nexera (Shimadzu Corporation) as the HPLC system under the following conditions to measure the fluorescence intensities.

### <HPLC conditions>

Column: ACQUITY UPLC BEH Glycan 1.7 µm, 2.1 × 150 mm
Solvent A: 50 mM aqueous solution of formic acid/ammonia (pH 4.4 )
Solvent B: acetonitrile
Gradient: solution A 25% (0 minutes) → solution A 42% (38.5 minutes)
Column temperature: 60°C→flow rate: 0.5 mL/minute
Ex: 280 nm, Em: 350 nm, Injection amount: 1 µL
As a result, the aoWR-NH2 exhibited a fluorescence intensity approximately 2.7 times as high as that of the aoWR (Figs. 1 and 2).

### (Example 2)

### (Release of N-Linked Sugar Chain from Glycoprotein)

To 40 mg of IgG from human serum (SIGMA, I4506), 2 mL of ultrapure water and 200 µL of each of a 1 M aqueous solution of ammonium bicarbonate (Wako Pure Chemical Industries, Ltd. , 017-02875) and a 120 mM aqueous solution of DTT (dithiothreitol, SIGMA, D9779) were added and allowed to react at 60°C for 30 minutes. After the reaction, 400 µL of a 123 mM aqueous solution of IAA (iodoacetamide, Wako Pure Chemical Industries, Ltd. , 093-02152) was added thereto and allowed to react under light-shielded condition at room temperature for 1 hour. Subsequently, a solution obtained by dissolving 16000 U of trypsin (SIGMA, T0303) in 400 µL of 1 mM hydrochloric acid was all added thereto and allowed to react at 37°C for 1.5 hours, thereby fragmentating the protein portion into peptides. The solution was heated at 90°C for 10 minutes to inactivate trypsin. Thereafter, the sugar chain was released from the peptides by the treatment with 80 U of peptide-N-glycosidaseF (Roche, 1-365-193). Thus, a sugar chain solution was obtained.

### (Labeling and Purification of N-linked sugar chain)

To a disposable column in which 5 mg of particles having a hydrazide group (BlotGlyco (R)), manufactured by Sumitomo Bakelite Co., Ltd., BS-45603) had been introduced as a carrier for capturing a sugar chain, 20 µL of the sugar chain solution and 180 µL of a 2% solution of acetic acid/acetonitrile were added and allowed to react at 80°C for 1 hour. The reaction was carried out in an open system. It was visually confirmed that the solvent was completely evaporated, and that the particles were in a dried, solid state.

The particles to which the N-linked sugar chain bound were washed with a 2 M aqueous solution of guanidine, water, and a 1% solution of triethylamine/methanol. Then, a 10% solution of acetic anhydride/methanol was added thereto and allowed to react at room temperature for 30 minutes, thereby capping unreacted hydrazide groups. After the capping, the beads were washed with 10 mM hydrochloric acid, methanol, and dimethyl sulfoxide.

Subsequently, the carboxyl group of the sialic acid residue was methyl esterified. Specifically, 100 µL of a 500 mM solution of 1-methyl-3-p-tolyltriazene (MTT) (Tokyo Chemical Industry Co., Ltd., M0641)/dimethyl sulf oxide was added and allowed to react at 60 ° C f or 1 hour. After the reaction, the resultant was washed with methanol and water.

Finally, the reactions were carried out, so that the sugar chain was cleaved from the particles and labeled. Specifically, 20 µL of an aqueous solution of the aoWR-NH2 and 180 µL of a 2% solution of acetic acid/acetonitrile were added and allowed to react at 80°C for 1 hour. After a humidification treatment (left standing in a powder form at a humidity of 100% at 37°C for 15 hours), ultrapure water was added to the aoWR-NH2 aqueous solution to adjust the aqueous solution to 20 mM. The reactions were carried out in an open system. It was visually confirmed that the solvent was completely evaporated, and that the particles were in a dried, solid state. To the dried beads, 50 µL of 2 mM hydrochloric acid was added to recover the N-linked sugar chain.

### (Removal of Excessive Labeling Reagents)

The recovered sugar chain solution was 10-fold diluted with acetonitrile, and then added to a silica column (included in the BlotGlyco kit), so that the labeled sugar chain was adsorbed to the silica gel. After the column was washed with acetonitrile, the labeled sugar chain was recovered using 50 µL of ultrapure water.

### (Comparative Example 1)

The purification was performed in the same manner as in Example 2, but aoWR was used as a label and 50 µL of ultrapure water was added to the dried beads to recover the N-linked sugar chain.

### (Comparative Example 2)

The purification was performed in the same manner as in Example 2, but aoWR (not humidified) was used as a label and 50 µL of ultrapure water was added to the dried beads to recover the N-linked sugar chain.

### (Analysis of Labeled Sugar Chain)

The recovered labeled sugar chain solutions were analyzed using MALDI-TOF MS (Autoflex III smartbeam, manufactured by Bruker Daltonics Inc.) in a reflector mode and a positive ion mode. As the matrix, 2,5-dihydroxybenzoic acid (Bruker, 201346) was used.

As apparent from the result shown in Fig. 3, when aoWR was humidified, contamination peaks appeared which were smaller than the original mass-to-charge ratio by approximately 14 presumably due to the hydrolysis of the methyl ester group. In contrast, no contamination peak appeared from the aoWR-NH2 in which the functional group was substituted with an amide group.

### [Industrial Applicability]

The present invention makes it possible to prepare a labeled sugar chain sample efficiently from a biological sample, and to efficiently detect the prepared labeled sugar chain sample by mass spectrometry and the like. Thus , the present invention is widely utilizable in the fields of tests and researches related to sugar chains, and also applicable to the fields of, for example, property analysis, quality control, and so forth of drugs and foods containing sugar chains.

## Claims

1. A compound represented by the following formula (1) : 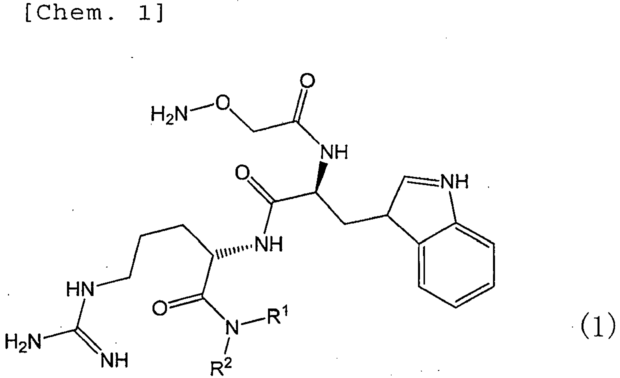 (R¹ and R² each independently represent any one of a hydrogen atom and an alkyl group).

2. The compound according to claim 1, wherein R¹ and R² in the formula (1) each independently represent any one of a hydrogen atom, a methyl group, and an ethyl group.

3. The compound according to claim 1 or 2, which is represented by the following formula:

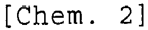

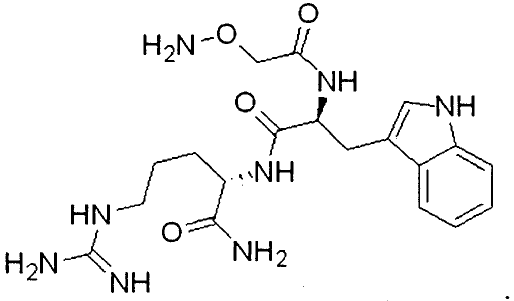

4. A labeling reagent comprising the compound according to any one of claims 1 to 3 as an effective component.

5. A method for preparing a labeled sugar chain sample, comprising the steps of:
(a) bringing a sample containing a sugar chain into contact with a carrier having any one of a hydrazide group and an aminooxy group as a functional group for capturing the sugar chain to cause an aldehyde group of the sugar chain to react with the any one of a hydrazide group and an aminooxy group of the carrier, thereby capturing the sugar chain on the carrier;
(b) washing the carrier capturing the sugar chain; and
(c) bringing the compound according to any one of claims 1 to 3 into contact with the carrier capturing the sugar chain to release the sugar chain from the carrier and simultaneously to bind the compound to the sugar chain.

6. The method according to claim 5, wherein the carrier is coated with a polymer substance having a crosslinked polymer structure represented by the following formula (2) : 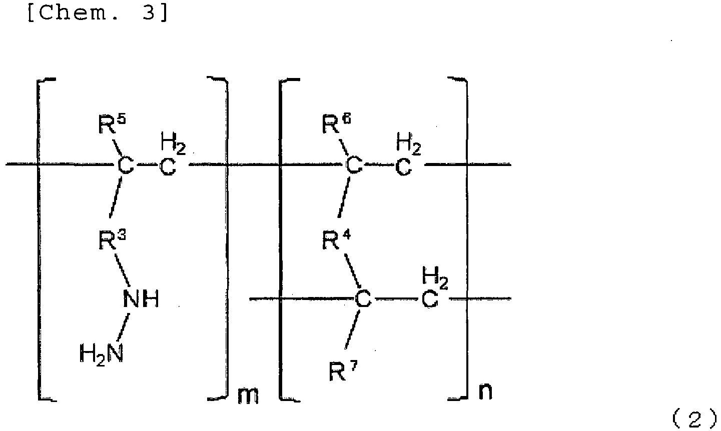 (R³ and R⁴ each independently represent a hydrocarbon chain having 1 to 20 carbon atoms which may be interrupted with any one of -O-, -S-, -NH-, -CO-, and -CONH-; R⁵, R⁶, and R⁷ each independently represent any one of H, CH₃, and a hydrocarbon chain having 2 to 5 carbon atoms; and m and n each independently represent the number of monomer units).

7. The method according to claim 6, wherein the carrier is coated with a polymer substance having a crosslinked polymer structure represented by the following formula (3): 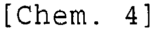 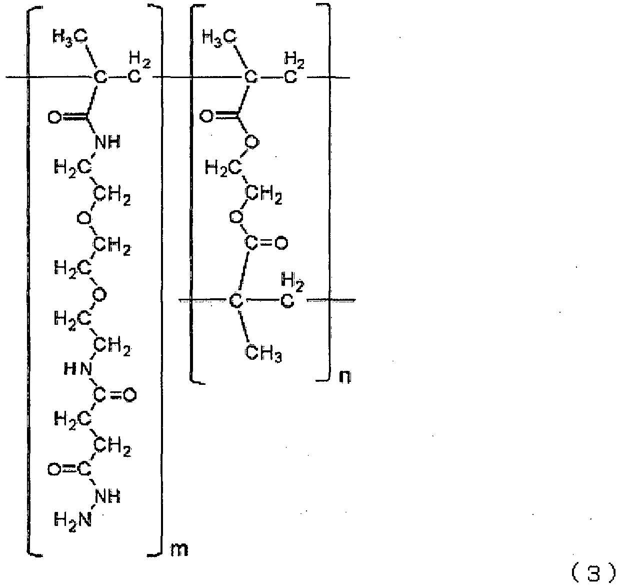 (m and n each independently represent the number of monomer units).

8. A method for analyzing a sugar chain, comprising the step of subjecting a labeled sugar chain sample prepared by the method according to any one of claims 5 to 7 to mass spectrometry to detect a sugar chain.
